# EUROPEAN PATENT APPLICATION

(11) **EP 3 199 949 A1**
(43) Date of publication of application: **02.08.2017**
(21) Application number: 16153221.3
(22) Date of filing: 28.01.2016
(51) Int. Cl.: G01N 33/574, A61K 39/00

(54) **METHOD OF DIAGNOSIS AND/OR PROGNOSIS AND/OR PREDICTION OF THERAPEUTIC RESPONSE OF NEOPLASTIC DISEASES**

(71) Applicant: Univerzita Palackého, 771 47 Olomouc (CZ)
(72) Inventor: VARANASI, Lakshman, 500062 Hyderabad (IN); HRUSKA, Miroslav, 022 01 Cadca (SK); DZUBAK, Petr, 751 03 Brodek u Prerova (CZ); HAJDUCH, Marian, 793 05 Moravsky Beroun (CZ)
(74) Representative: Hartvichova, Katerina

(57) **Abstract**

The invention provides a method of in-vitro determination of diagnosis and/or prognosis and/or prediction of therapeutic response of a neoplastic disease or a pre-neoplastic diasease from a sample taken from a body of a patient, said method comprising a step of determination of presence, and optionally amount, of a peptide containing a 9 amino acid sequence SATVNLTVI, said peptide being optionally post-translationally modified, in said sample, and subsequently a step of making the diagnosis and/or prognosis and/or prediction of therapeutic response. The peptide is a specific marker indicative of the presence of the IGSF5 protein.

## Description

### Field of Art

The present invention relates to a method of diagnosis and/or prognosis and/or prediction of therapeutic response of neoplastic diseases.

### Background Art

IGSF5 (Also called JAM4) occurs in tight junctions and the apical membrane of the epithelium in the colon and in glomerular cells of the kidney. IGSF5 (or JAM4) is a transmembrane protein that occurs in the apical membrane of epithelial cells. Immunohistochemistry of various healthy human tissues shows that the protein is expressed at low levels, if at all (http://www.proteinatlas.org/ENSG00000183067-IGSF5/tissue/primary+data). This is ascribed to the unreliability of the antibody used. There is more expression of IGSF5 in various cancer cell lines but the results are again uncertain because of the antibody (the cell-lines CaCO2, Capan and HepG2 yield scores of 6, 7 and 11 respectively on an antibody staining score scale of 1-1000, http://www.proteinatlas.org/ENSG00000183067-IGSF5/cell/HPA057282). Cancer tissues stain negative (http://www.proteinatlas.org/ENSG00000183067-IGSF5/cancer).

The immunohistochemistry for the tissues was so far performed using a polyclonal rabbit antibody, HPA057282, available from Sigma-Aldrich (http://www.proteinatlas.org/ENSG00000183067-IGSF5/antibody). However, the antigenic amino acid sequence for this antibody does not include the peptide reported in this application.

### Disclosure of the Invention

The present invention provides a method of in-vitro determination of diagnosis and/or prognosis and/or prediction of therapeutic response of a neoplastic disease or a pre-neoplastic diasease from a sample taken from a body of a patient, said method comprising a step of determination of presence, and optionally amount, of a peptide that is indicative of presence of IGSF5, said peptide being optionally post-translationally modified, in said sample, and subsequently a step of making the diagnosis and/or prognosis and/or prediction of therapeutic response.

More particularly, the present invention provides a method of in-vitro determination of diagnosis and/or prognosis and/or prediction of therapeutic response of a neoplastic disease or a pre-neoplastic diasease from a sample taken from a body of a patient, said method comprising a step of determination of presence, and optionally amount, of a peptide containing a 9 amino acid sequence SATVNLTVI (SEQ ID NO. 1), said peptide being optionally post-translationally modified, in said sample, and subsequently a step of making the diagnosis and/or prognosis and/or prediction of therapeutic response.

The peptide may preferably contain from 9 to about 100 amino acids, or from 9 to about 70 amino acids, or from 9 to about 50 amino acids.

The detected peptide in particular contains the 9 amino acid sequence SATVNLTVI, preferably it contains a 10 amino acid sequence SATVNLTVIR (SEQ ID NO. 2) or SATVNLTVIW (SEQ ID NO. 3). The other amino acids in the peptide correspond to the sequences adjacent in the IGSF5 protein to the sequence SATVNLTVI (amino acids 227-235 of IGSF5).

Post-translational modification may include, for example, glycosylation and/or phosphorylation.

The peptide is based on a 10 amino acid sequence (N-term-SATVNLTVIR-C-term) spanning amino acids 227-236 of the Immunoglobulin Superfamily 5 protein (IGSF5). This sequence occurs in the extramembrane, extracellular portion of the protein (said portion being formed by amino acids 1-266). It is glycosylated at N231 and has a predicted phosphorylation site at T233 (http://www.phosphosite.org/proteinAction?id=14739002&showAllSites=true). The sequence's predicted location is on the surface of the extracellular domain of the protein (PSI, Protein Model Portal, Q9NSI5: 3D Model From MODBASE Based On 2ensA -- Protein Model Portal - PSI SBKB. at <http://www.proteinmodelportal.org/?pid=modelDetail&provider=MODBASE&template= 2ensA&pmpuid=1000818659847&range_from=1&range_to=407&ref_ac=Q9NSI5&mapp ed_ac=Q9NSI5&zid=async>). The C-terminal amino acid in the sequence, R236 has been observed to be altered to W (Tryptophan) in non-gastrointestinal cancers. This is a confirmed somatic mutation, COSMIC mutation ID is COSM275551 that has been denoted as "potentially damaging" (BioMuta 2 (HIVE)).
The sequence is unique to the human protein and is not generated from any other protein in serum from mice xenografted with GI cancer cells. In other words, it is selectively representative of one protein, IGSF5, and cannot be confused with a different protein. The sequence is not an artefact and identifies the protein confidently.
It was discovered that said peptide is detectable in samples from patients suffering from a neoplastic disease, and is not detectable in samples from healthy individuals.

In further text, the term "neoplastic disease" is meant to include both "neoplastic disease" and "pre-neoplastic disease".

The neoplastic disease may preferably be an epithelial cancer, such as for example colon carcinoma, oesophageal carcinoma, oesophageal squamous cell carcinoma, gastric adenocarcinoma, gastric carcinoma, hepatocellular carcinoma, pancreatic carcinoma, lung carcinoma, breast carcinoma, prostate carcinoma. The neoplastic disease may also preferably be melanoma.

The sample taken from the body of a patient may preferably be a sample of tumor tissue or body fluid, such as urine, cerebrospinal fluid, ascites, effusions, systemic blood, serum or plasma.

The determination of presence and/or amount of the peptide may be performed using any suitable analytical method. In particular, liquid chromatography, mass spectrometry, immunochemical methods such as ELISA (Enzyme Linked Immunosorbent Assay), or combinations thereof may be used.

The ELISA method may be carried out in various formats, such as a direct ELISA assay, an indirect ELISA assay, a Sandwich ELISA assay or any other suitable format. For example, in sandwich ELISA, the peptide of the invention constitutes the epitope for the detection antibody. A second peptide, from a spatially distinct region of the parent protein, constitutes the epitope for the capture antibody. The capture antibodies can be pre-coated in assay plate wells and bind to the (parent) protein in applied patient sample. The detection antibody binds to the peptide epitope and is in turn recognized by a secondary antibody that is linked to an enzyme. Presence of the protein in patient blood is indicated by known methods, such as colorimetry, fluorimetry or chemiluminescence, when said enzyme is supplied with a substrate.

In a preferred embodiment, a step comprising digestion of proteins in the sample and/or a step of fractionation of the proteins and/or peptides present in the sample or in the digested sample is carried out prior to the step of determination of presence and/or amount of the peptide. Fractionation may be performed by various methods, such as electrophoresis, or the stage-tip method (Wiśniewski, J. R., Zougman, A., Nagaraj, N. & Mann, M. Universal sample preparation method for proteome analysis. Nat. Methods 6, 359-362 (2009))..

Determination of diagnosis includes identification of the presence of a neoplastic disease, monitoring the activity of the neoplastic disease, and/or monitoring of minimal residual disease.

Determination of prognosis includes determination of the developmental stage of the neoplastic disease.

Making of diagnosis may in a preferred embodiment be based on the presence (detection) in the sample of the peptide of the invention being indicative of presence of a neoplastic disease, while the absence (non-detection) of the peptide is indicative of no neoplastic disease present. In another embodiment, detection in the sample of the amount of the peptide of the invention above a threshold value is indicative of presence of a neoplastic disease, while the amount of the peptide being under said threshold value is indicative of no neoplastic disease present. The threshold value may be obtained by statistical analysis of data for groups of healthy individuals and groups of patients with diagnosed neoplastic disease.

Making of prognosis may in a preferred embodiment be based on the correlation of the detected amount of the peptide of the invention with a stage of a neoplastic disease. The threshold values of peptide amounts for individual stages of the neoplastic disease may be obtained by statistical analysis of data for groups of patients with diagnosed neoplastic disease in said individual stages. The stages of the neoplastic disease may include temporal stages or disease progression/developmental stages.

Making of prediction of therapeutic response may in a preferred embodiment be based on the correlation of the detected amount of the peptide of the invention with therapeutic response. The threshold values of peptide amounts for various types of therapeutic response to the treatment of the neoplastic disease with relevant medicaments and/or therapeutic regimes may be obtained by statistical analysis of data for groups of patients with diagnosed neoplastic disease and showing said types of therapeutic response. The types of therapeutic response may include responsiveness, resistance, or partial responsiveness to the relevant medicament or therapeutic regime.

The amount of the peptide of the present invention may preferably be normalized. Normalization is commonly carried out by determining the amount of said peptide in parallel with the determination of the amount of normalization marker which is a peptide, protein or nucleic acid sequence, typically a peptide, protein or nucleic acid sequence the amount of which in the given tissue is constant and remains unaffected by the neoplastic disease.

Preferably, measurement of absolute or relative quantities of peptide in the sample may be carried out. The measurement may be done by a targeted quantitation technique, such as Selected Reaction Monitoring (SRM), Multiple Reaction Monitoring (MRM), Parallel Reaction Monitoring (PRM) and/or Single Ion Monitoring (SIM).

The described embodiments for making of diagnosis, prognosis and prediction of therapeutic response may be freely combined according to the needs and consideration of a clinician and/or scientist.

The present invention further includes use of the peptide indicative of presence of IGSF5, said peptide being optionally post-translationally modified, for a method of in-vitro determination of diagnosis and/or prognosis and/or prediction of therapeutic response.

More particularly, the present invention further includes use of the peptide containing a 9 amino acid sequence SATVNLTVI, said peptide being optionally post-translationally modified, for a method of in-vitro determination of diagnosis and/or prognosis and/or prediction of therapeutic response.

The present invention further includes use of the peptide indicative of presence of IGSF5, said peptide being optionally post-translationally modified, for production of monoclonal/polyclonal antibodies. Such antibodies may then be used in diagnostic devices in the method of the present invention.

More particularly, the present invention further includes use of the peptide containing a 9 amino acid sequence SATVNLTVI, said peptide being optionally post-translationally modified, for production of monoclonal/polyclonal antibodies. Such antibodies may then be used in diagnostic devices in the method of the present invention.

### Examples of carrying out the Invention

### Culture of cell lines/patient tumors:

Seventeen of the cell-lines used in this study were procured from DSMZ, Germany, and three more from the American Type Culture Collection (ATCC). All cell-lines were cultured in the laboratory according to the vendors' instructions. Tumors derived from a few patients undergoing treatment were also used as patient-derived xenografts (PDXs).

### Xenograft of cell lines and patient tumors:

The different cancer cells were harvested from their culture flasks/dishes, spun down in a 15 or 50ml conical-bottom tube at 4 °C at 350g. Cells were then suspended in tissue culture medium (RPMI) and mixed with matrigel.

The transport medium with tumor tissue (excised from patients) was transferred to a stainless steel strainer. Tissue was cut into small pieces (about 0.3 mm³), forced through the strainer using a syringe piston and collected in a Petri dish. The cell-suspension was transferred to a Falcon tube and 20ml of media added. The cell suspension was centrifuged at 600g for 7 minutes. The supernatant was discarded, the cells resuspended in an appropriate volume of medium and mixed with matrigel. 0.5-1*10⁶ cells in 100 µl appear to be optimal.

SCID mice were purchased from Harlan Laboratories (now renamed Envigo). Each mixture of cell-lines/patient tumor cells with matrigel was injected into five SCID mice, with each mouse receiving two xenografts (subcutaneously, one in each flank). 2.5*10⁶ - 5*10⁶ cells were used for each xenograft. The xenografts were allowed to grow until they reached an approximate size of 1 cm³. Mice were sacrificed by dislocation of the cervical spine, under anaesthesia (plexus axillaris in general inhalation anesthesia), exsanguinated, and the blood and tumors collected. All animal procedures were in compliance with Czech and European laws and regulations governing the transport, housing and use of animals in research.

Blood from tumor bearing animals was processed into serum within 60 minutes of sampling. Blood was allowed to stand on ice for 15-30 minutes to allow it to clot. It was then spun at 840g for 10 minutes at 4°C to separate the serum from the clot. Serum was then aliquoted into 100 microliter fractions and stored at -80 °C.

### Detection of the N-Glycosylated peptides

### Solid-phase extraction of N-glycopeptides (SPEG):

Protocol adapted from Tian, Y., Zhou, Y., Elliott, S., Aebersold, R. & Zhang, H. Solid-phase extraction of N-linked glycopeptides. Nat. Protoc. 2, 334-339 (2007); Zhang, Y., Kuang, M., Zhang, L., Yang, P. & Lu, H. An accessible protocol for solid-phase extraction of N-linked glycopeptides through reductive amination by amine-functionalized magnetic nanoparticles. Anal. Chem. 85, 5535-5541 (2013).

The protein concentration of the serum sample was measured. If the sample concentration is more than 5 mg/ml it is diluted 10-fold in denaturing buffer. If the sample concentration is less than 5 mg/ml solid urea, ammonium bicarbonate and 10% SDS solution is added directly to the sample to prepare a denaturing buffer containing 8 M urea 0.4 M Ammonium bicarbonate and 0.1% SDS. Samples were sonicated for 1 minute at room temperature (with a probe sonicator) using a duty cycle of 0.5 and a power output of 35%. Approximately 1mg of protein was used for the SPEG protocol

TRIS-phosphine was added to the sample to a final concentration of 10mM and samples incubated at 60 °C for 60 min. This was followed by the addition of Iodoacetamide to a final concentration of 12mM and incubation at room temperature (20°C) in the dark for 30 min. The sample was diluted with Pottassium Phosphate buffer (100 mM pH 8.0) to reduce the urea concentration to less than 2M. The sample was then digested with Trypsin for 16 hours at 37°C, with agitation, at a concentration of 1 ug of Trypsin per 200 ug of serum protein.

The digested peptide mixture was acidified using 5 N HCl until the pH dropped to 3. A C-18 SepPak column was conditioned by washing the column twice with 1 ml of 50 % ACN in 0.1 % TFA and then twice with 1ml 0.1 % TFA. The sample was loaded onto the conditioned C-18 SepPak column and the C-18 SepPak column washed three times with 1ml of 0.1%TFA. The peptides were eluted twice with 0.2 ml of 50 % ACN in 0.1 % TFA. Sodium periodate was then added to a final concentration of 10mM, to each sample, and the samples incubated in the dark at 4 °C for 1 hour. They were diluted 10-fold in 0.1% TFA to stop the reaction. A C-18 SepPak column was conditioned by washing the column twice with 1 ml of 50 % ACN in 0.1 % TFA and then twice with 1ml 0.1 % TFA. The sample was loaded onto the conditioned C-18 SepPak column and the C-18 SepPak column washed three times with 1ml of 0.1 % TFA. The peptides were eluted twice with 0.2 ml of 80 % ACN in 0.1 % TFA. 25 µl of hydrazide resin (50 µl of 50 % slurry) was readied for every sample. The resin was spun briefly in a microfuge tube (2500 g for 30-60 s) and the supernatant removed. The hydrazide resin was washed in 250 ml of water three times. The resin was spun briefly each time (2500 g for 30-60 s) and the water removed from the resin. The oxidized peptides were added to the hydrazide resin and the reaction mixture incubated at room temperature for 16 hours with agitation.

The resin was spun down(2500 g for 30-60 s) and the supernatant removed. The resin was washed four times with 250 µl of Ammonium bicarbonate buffer and resuspended in 25 µl of Ammonium Bicarbonate buffer. 3µl of PNGase F (500units per µl, New England Biolabs) was added and the digestion incubated at 37 °C for 4 hours with agitation. The resin was spun down (2500g for 30-60s) and the supernatant collected. It was washed twice with 100µl of Ammonium Bicarbonate buffer and the washes pooled with the supernatant. The pooled sample contained the isolated N-glycopeptides.

### Liquid chromatography and LC-MS/MS

Samples isolated from SPEG were acidified using HCl acid and repurified on the SepPak C18 columns. The samples were then evaporated to dryness in a Speed Vac (Thermo) and resuspended in 100 microlitres of 5% Acetonitrile, 0.1% Formic acid for LC-MS/MS analysis.

Screening of serum samples, or discovery analysis was performed on an Orbitrap Elite instrument (Thermo) instrument fitted with a Proxeon Easy-Spray ionization source (Thermo), coupled to an Ultimate 3000 RSLCnano chromatograph. Sample was injected into the spectrometer via a Proxeon Easy-Spray ionization source (Thermo). Ten microliters of the above sample were loaded on a PepMap 100 (75 µm x2 cm, 3 µm, 100 Å pore size) desalting column (Thermo) "in-line" with a PepMap RSLC (75 µm x 15 cm, 3 µm, 100 Å pore size) analytical column (Thermo) heated to 35°C. Peptides were separated on the analytical column by ramping the organic phase from 5 % to 35 % at a constant flow rate of 300 nl per minute over 150 minutes. The aqueous (A) and organic phase reagents (B) are, respectively, 0.1 % Formic acid and 80 % Acetonitrile in 0.1 % Formic acid.

Data was collected using a top-20 method. The MS¹ survey scans were performed in the ion-trap in the positive ion mode at a source voltage of 2000 V. Precursor ions with charges 2-6 were selected and scanned across an m/z range of 300- 1500 at a resolution of 120,000 full width at half maximum (FWHM) nominal resolution settings and a cycle time of 3 seconds. The precursor mass window was set at 10 ppm. Data was acquired in the profile mode. The twenty most intense ions in the survey scan were selected for fragmentation by collision-induced dissociation (CID) in the Orbitrap, using a normalized collision energy (CE) of 30 eV. The same ions were dynamically excluded from reselection for 40 seconds. An automatic gain control target of 2.0e5 ions and an accumulation time of 50 ms was applied. MS² spectra were acquired in the Orbitrap in the normal scan mode using a precursor m/z selection window of 1.6 Th. An automatic gain control of 1000 ions and an injection time of 50 ms was used. Data was acquired in profile mode. Three biological replicates were analyzed for each of the cell-line xenografts and five for the control. Samples from patient tumor xenograft samples were available as one, two or three biological replicates and were analyzed as such.

A second discovery run was also performed using High-energy collisional dissociation (HCD). Precursor ions were scanned across an m/z range of 400-1600. MS² was performed in the Orbitrap with an HCD collision energy of 28% at a resolution of 15000 FWHM and an automatic gain of control target count of 50000. MS² data was acquired in centroid mode. The other acquisition parameters were as described for the CID run.

### Computational analysis of tandem MS data

### Matching of peptides and spectra:

The computational system identifies peptides using database search approach of proteotypic peptides of reference proteins for human and mouse. Human and murine databases of proteins were downloaded (UniProt) and proteins were in-silico digested (trypsin) into peptides. Fragmentation spectra for tryptic peptides in the database were predicted and these spectra were compared to the experimental spectra (X!Tandem). Due to biochemical protocol used in the preparation of the samples, additional changes were made in prediction of fragmentation spectra. Each Cysteine was assumed to be carbamidomethylated (fixed modification); Methionines were assumed to be potentially oxidized and Asparagines potentially deamidated (variable modifications). The similarity between experimental and predicted fragmentation spectra was calculated (X!Tandem's HyperScore) and statistically significant matches were selected (E-Value < 0.1).

### Post-search analysis:

Statistically significant matches were further evaluated with respect to conformance to PNGaseF cleavage motif and their exclusive assignability to human proteome. Only deamidated peptides were selected (glycopeptides before PNGaseF cleavage). From those, only peptides with PNGaseF cleavage motif (NX[S/T], where X is any amino acid other than proline) were retained. The peptide sequences were then analyzed for their exclusive presence in human reference database. Peptides present in mouse database, or both mouse and human database were removed from the results. The procedure should preserve exclusively human peptides; moreover, mouse controls were used in order to avoid potential identification artefacts.

### Detection of non-glycosylated peptides:

The plasma or serum samples are processed to generate and identify non-glycosylated form(s) of the peptide. The same is quantified as described above for glycosylated peptides.

### Tryptic digestion of sample and fractionation for LC-MS

Samples are processed using a *bottom*-*up* proteomics approach, including in-solution digestion and the Filter-aided sample preparation protocol (FASP). The process comprises the following steps.

The sample is denatured by an 8.0 M urea solution containing 0.1% SDS and 0.2M Tris-HCl, pH 8.5 and treated with 5 mM DTT at 37 °C for 1 hour to reduce and cleave the disulfide bonds in the protein. A 25 mM iodoacetamide solution is used to alkylate the reactive sulfhydryls and render them unreactive. The reaction is performed at room temperature, in the dark, for 30 minutes. The sample is diluted in 0.2 M Tris-HCl buffer, pH 8.5 to lower the urea concentration to below 2 M. It is incubated with trypsin at a protein: protease ratio of 100:1, at 37 °C for approximately 16 hours, to digest sample proteins into their constituent peptides. The resulting peptide mixture is purified using a C-18 reverse phase column (as described above) and resuspended in sample buffer for analysis by LC-MS (as described above).

### Fractionation of sample and preparation for LC-MS

Because plasma and serum are complex mixtures of proteins, the purified peptide digest is fractionated. Fractionation is performed electrophoretically, or by the stage-tip method.

Tables 1 and 2 show the observed presence or absence of the peptide SATVNLTVIR or SATVNLTVIW, respectively, in the tested samples.

**Table I**

| *Cell-line* | *Cancer type* | *Supplier* | *Peptide observed?* |
|---|---|---|---|
| CL-11 | Colon carcinoma | DSMZ | Yes |
| COLO-320 | Colon carcinoma | DSMZ | Yes |
| LOVO | Colon carcinoma | DSMZ | Yes |
| LS-174T | Colon carcinoma | DSMZ | Yes |
| FLO-1 | Oesophageal carcinoma | DSMZ | Yes |
| OE-19 | Oesophageal carcinoma | DSMZ | Yes |
| KYSE-30 | Oesophageal squamous cell carcinoma | DSMZ | Yes |
| KYSE-70 | Oesophageal squamous cell carcinoma | DSMZ | Yes |
| 23132-87 | Gastric adenocarcinoma | DSMZ | Yes |
| MKN-45 | Gastric adenocarcinoma | DSMZ | Yes |
| NCI-N87 | Gastric carcinoma | DSMZ | Yes |
| HEP-3B | Hepatocellular carcinoma | DSMZ | Yes |
| HEP-G2 | Hepatocellular carcinoma | DSMZ | Yes |
| BXPC-3 | Pancreatic carcinoma | ATCC | Yes |
| JOPACA-1 | Pancreatic carcinoma | ATCC | Yes |
| YAPC | Pancreatic carcinoma | ATCC | Yes |

**Table II**

| *Patient derived xenografts* | *Cancer Type* | *Peptide observed?* |
|---|---|---|
| 2 | Gastric | Yes |
| 2 | Gastric | Yes |
| 2 | Gastric | Yes |
| 3 | Gastric | Yes |
| 3 | Gastric | Yes |
| 4 | Ascites, Gastric | Yes |
| 10 | Gastric | Yes |
| 12 | Pancreatic | Yes |
| 12 | Pancreatic | Yes |
| 12 | Pancreatic | Yes |
| CON | Control SCID mice sera | No |
| CON | Control SCID mice sera | No |
| CON | Control SCID mice sera | No |
| CON | Control SCID mice sera | No |
| CON | Control SCID mice sera | No |

## Claims

1. A method of in-vitro determination of diagnosis and/or prognosis and/or prediction of therapeutic response of a neoplastic disease or a pre-neoplastic disease from a sample taken from a body of a patient, said method comprising a step of determination of presence, and optionally amount, of a peptide indicative of presence of IGSF5, said peptide being optionally post-translationally modified, in said sample, and subsequently a step of making the diagnosis and/or prognosis and/or prediction of therapeutic response.

2. The method of in-vitro determination of diagnosis and/or prognosis and/or prediction of therapeutic response of a neoplastic disease or a pre-neoplastic diasease from a sample taken from a body of a patient according to claim 1, said method comprising a step of determination of presence, and optionally amount, of a peptide containing a 9 amino acid sequence SATVNLTVI, said peptide being optionally post-translationally modified, in said sample, and subsequently a step of making the diagnosis and/or prognosis and/or prediction of therapeutic response.

3. The method according to claim 1 or 2, wherein the neoplastic disease is an epithelial cancer or melanoma.

4. The method according to any one of claims 1 to 3, wherein the sample taken from the body of a patient is a sample of tumor tissue or body fluid, such as urine, cerebrospinal fluid, ascites, effusions, systemic blood, serum or plasma.

5. The method according to any one of claims 2 to 3, wherein the peptide contains sequence SATVNLTVIR or SATVNLTVIW.

6. The method according to any one of claims 1 to 5, wherein the peptide contains from 9 to 100 amino acids, or from 9 to 70 amino acids, or from 9 to 50 amino acids.

7. The method according to any one of claims 1 to 6, wherein a step comprising digestion of proteins in the sample and/or a step of fractionation of the proteins and/or peptides present in the sample or in the digested sample is carried out prior to the step of determination of presence and/or amount of the peptide.

8. The method according to any one of claims 1 to 7, wherein making of diagnosis is based on the presence in the sample of the peptide being indicative of presence of a neoplastic disease.

9. The method according to any one of claims 1 to 7, wherein making of diagnosis is based on the detection in the sample of the amount of the peptide of the invention above a threshold value being indicative of presence of a neoplastic disease.

10. The method according to any one of claims 1 to 7, wherein making of prognosis is based on the correlation of the detected amount of the peptide of the invention with a stage of a neoplastic disease.

11. The method according to any one of claims 1 to 7, wherein making of prediction of therapeutic response is based on the correlation of the detected amount of the peptide of the invention with therapeutic response.

12. Use of the peptide indicative of presence of IGSF5, said peptide being optionally post-translationally modified, for a method of in-vitro determination of diagnosis and/or prognosis and/or prediction of therapeutic response.

13. Use according to claim 12 of the peptide containing a 9 amino acid sequence SATVNLTVI, said peptide being optionally post-translationally modified, for a method of in-vitro determination of diagnosis and/or prognosis and/or prediction of therapeutic response.

14. Use of the peptide indicative of presence of IGSF5, said peptide being optionally post-translationally modified, for production of antibodies.

15. Use according to claim 14 of the peptide containing a 9 amino acid sequence SATVNLTVI, said peptide being optionally post-translationally modified, for production of antibodies.


SEQUENCE LISTING

<110>  Univerzita Palackeho v Olomouci

<120>  Method of diagnosis and/or prognosis and/or prediction of
       therapeutic response of neoplastic diseases

<130>  P1465EP00

<160>  3

<170>  PatentIn version 3.5

<210>  1
<211>  9
<212>  PRT
<213>  Homo sapiens

<400>  1

Ser Ala Thr Val Asn Leu Thr Val Ile
1               5


<210>  2
<211>  10
<212>  PRT
<213>  Homo sapiens

<400>  2

Ser Ala Thr Val Asn Leu Thr Val Ile Arg
1               5                   10


<210>  3
<211>  10
<212>  PRT
<213>  Homo sapiens

<400>  3

Ser Ala Thr Val Asn Leu Thr Val Ile Trp
1               5                   10